(19) Europäisches Patentamt
European Patent Office
Office européen des brevets

(11) EP 1 689 731 B1

(12) **EUROPÄISCHE PATENTSCHRIFT**

(45) Veröffentlichungstag und Bekanntmachung des Hinweises auf die Patenterteilung:
**07.03.2007 Patentblatt 2007/10**

(21) Anmeldenummer: **04790531.0**

(22) Anmeldetag: **16.10.2004**

(51) Int Cl.:
***C07D 307/86*** *(2006.01)* ***A61K 31/34*** *(2006.01)*
***A61P 31/04*** *(2006.01)* ***A61P 31/10*** *(2006.01)*
***C12R 1/66*** *(2006.01)*

(86) Internationale Anmeldenummer:
**PCT/EP2004/011695**

(87) Internationale Veröffentlichungsnummer:
**WO 2005/047275 (26.05.2005 Gazette 2005/21)**

(54) **2-PHENYL-BENZOFURAN-DERIVATE, VERFAHREN ZUR IHRER HERSTELLUNG UND IHRE VERWENDUNG**

2-PHENYL-BENZOFURAN DERIVATIVES, METHOD FOR THE PRODUCTION THEREOF AND THEIR USE

DERIVES DE 2-PHENYL-BENZUFURANE, PROCEDE PERMETTANT DE LES PRODUIRE ET UTILISATION CORRESPONDANTE

(84) Benannte Vertragsstaaten:
**AT BE BG CH CY CZ DE DK EE ES FI FR GB GR HU IE IT LI LU MC NL PL PT RO SE SI SK TR**

(30) Priorität: **31.10.2003 DE 10351315**

(43) Veröffentlichungstag der Anmeldung:
**16.08.2006 Patentblatt 2006/33**

(73) Patentinhaber: **Sanofi-Aventis Deutschland GmbH**
**65929 Frankfurt am Main (DE)**

(72) Erfinder:
- **VERTESY, Laszlo**
  **65817 Eppstein-Vockenhausen (DE)**
- **KURZ, Michael**
  **65926 Frankfurt am Main (DE)**
- **MARKUS-ERB, Astrid**
  **65835 Liederbach (DE)**
- **TOTI, Luigi**
  **65239 Hochheim (DE)**

(56) Entgegenhaltungen:
**WO-A-02/10156** **GB-A- 2 386 891**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 689 731 B1

Printed by Jouve, 75001 PARIS (FR)

**Beschreibung**

Zur Behandlung von bakteriellen Infektionskrankheiten wird eine große Zahl von Antibiotika therapeutisch eingesetzt. Die Krankheitserreger werden aber zunehmend resistent gegen die verwendeten Arzneimittel, es droht sogar eine große Gefahr durch sogenannte multiresistente Keime, die nicht nur gegen einzelne Antibiotika-gruppen, wie z. B. β-Lactam-Antibiotika, Glycopeptide oder Macrolide widerstands-fähig geworden sind, sondern gleichzeitig mehrere Resistenzen tragen. Es gibt sogar Krankheitserreger, die gegen alle im Handel erhältlichen Antibiotika resistent geworden sind. Infektionskrankheiten, die durch solche Keime verursacht werden, sind nicht mehr therapierbar. Deshalb gibt es einen großen Bedarf an neuen Mitteln, die gegen resistente Keime eingesetzt werden können. Es sind zwar in der Literatur viele Tausend Antibiotika beschrieben worden, die meisten sind jedoch zu toxisch, um als Arzneimittel eingesetzt werden zu können.

Die Zellwand gram-positiver und gram-negativer Bakterien besteht zu einem Großteil aus Peptidoglycan (Murein), das als sogenannter Mureinsacculus die Zelle voll-ständig umschließt und ihr mechanische Stabilität verleiht sowie ihre morpho-logische Gestalt mitbestimmt. Peptidoglycan ist ein Makromolekül, das sich aus den 1,4-β-glykosidisch verknüpften Aminozuckern N-Acetylglucasamin und N-Acetyl-muraminsäure in alternierender Reihenfolge zusammengesetzt. Durch Querver-netzungen über kurze Peptidbrücken erhalten die Zuckerketten eine hohe Stabilität. Die Biosynthese von Peptidoglycan wird durch eine Reihe von im Zytoplasma gelöster sowie membrangebundener Enzyme katalysiert. Viele dieser Enzyme sind für Bakterien spezifisch und repräsentieren ideale Angriffspunkte bei der Suche nach neuen Antibiotika.

Die bifunktionale bakterielle N-Acetyl-glucosamin-1-phosphat-uridyltransferase (GlmU) katalysiert die Bildung von UDP-N-Acetyl-glucosamin in einer Zweistufen-Reaktion aus Glucosamin-1-phosphat. UDP-N-Acetyl-glucosamin ist ein fundamentaler Baustein der bakteriellen Zellwand-Biosynthese, und die Hemmung seiner Bildung ist daher ein Erfolg versprechender Weg zur Auffindung von neuartigen antibakteriellen Therapeutika (Sulzenbacher et al., J. Biol. Chem. 2001, 276 (15), 11844-11851).

Es wurden bereits Verbindungen aus Kulturen des *Aspergillus flavipes* beschrieben, wie z. B. das Flavipin (Raistrick et al., Biochem. J. 1956, 63, 395) als Inhibitor der Atmungskette, und das Phytotoxin Flavipucin (Findlay et al., J.C.S. Perkin I 1972, 2071) oder die Verbindung F-90558 (Kuraya et al., JP 20012862292 A2) als Antitumormittel. Pyrimidin-2-ylamin-substituierte Phenyl-benzofurane sind beispielsweise in Burri et al., WO 02/10156 beschrieben.

Es wurde überraschend gefunden, dass der Stamm *Aspergillus flavipes* ST003878 (DSM 15290) DSMZ-Deutsche Sammlung von Mikroorganismen und Zellkulturen GmbH, eingangsnummer DSM 15 290 neuartige Antibiotika zu bilden vermag, die wirksame Hemmstoffe der GlmU sind und sich als Leitstrukturen für weiteren antibakteriell wirksame Agenzien eignen.

Gegenstand der Erfindung ist eine Verbindung der Formel (I)

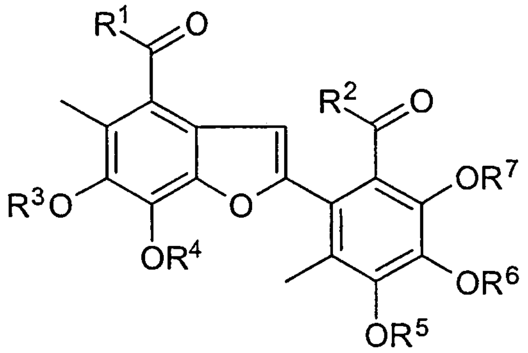

(I)

wobei

$R^1$ und $R^2$ unabhängig voneinander H, OH oder -O-($C_1$-$C_6$)-Alkyl, und $R^3$, $R^4$, $R^5$, $R^6$ und $R^7$ unabhängig voneinander H, -($C_1$-$C_6$)-Alkyl oder -C(O)-($C_1$-$C_6$)-Alkyl bedeuten,

oder ein physiologisch verträgliches Salz der Verbindung der Formel (I).

($C_1$-$C_6$)-Alkyl bedeutet eine geradkettige oder verzweigte Kohlenwasserstoffgruppe mit 6 Kohlenstoffatomen, beispielsweise Methyl (Me), Ethyl, n-Propyl, iso-Propyl, tert-Butyl oder n-Hexyl, vorzugsweise Methyl.

Vorzugsweise sind $R^1$ und $R^2$ in der Verbindung der Formel (I) gleich H.

Ferner bevorzugt sind $R^3$, $R^4$, $R^5$, $R^6$ und $R^7$ in der Verbindung der Formel (I) unabhängig voneinander gleich H oder Methyl.

Besonders bevorzugt sind $R^1$ und $R^2$ in der Verbindung der Formel (I) gleich H und $R^3$, $R^4$, $R^5$, $R^6$ und $R^7$ sind

unabhängig voneinander gleich H oder Methyl.

Ferner betrifft die Erfindung eine Verbindung der Formel (I), gekennzeichnet durch eine Verbindung der Formel (II)

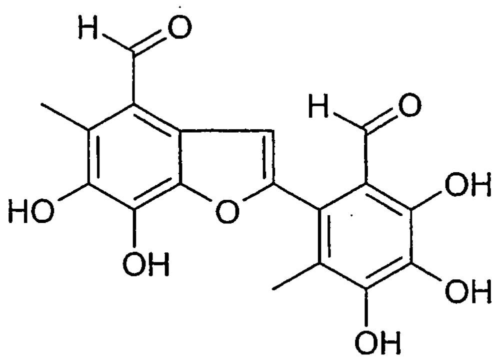

(II),

eine Verbindung der Formel (III)

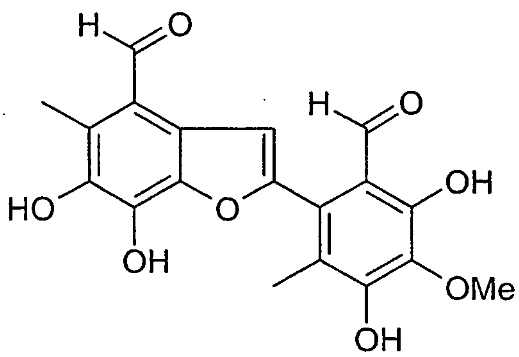

(III),

eine Verbindung der Formel (IV)

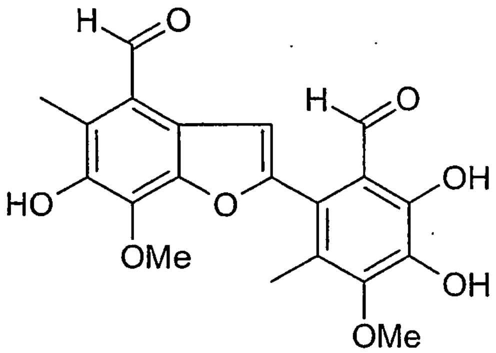

(IV),

und eine Verbindung der Formel (V)

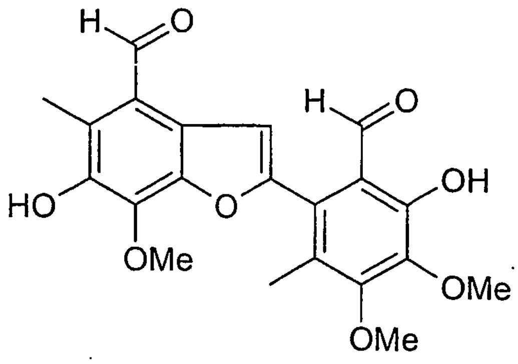

(V).

Unter physiologisch verträglichen Salzen von Verbindung der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z. B. Essigsäure, Zitronensäure, Benzoesäure, Maleinsäure, Fumarsäure, Weinsäure und p-Toluolsulfonsäure bevorzugt.

Die erfindungsgemäßen Verbindungen der Formel (I) besitzen keine Verwandtschaft mit herkömmlichen Antibiotika, wie zum Beispiel den β-Lactamen (Penicilline, Cephalosporine), Aminoglycosiden (Streptomycin), Makroliden (Erythromycin), Chinolonen (Ciprofloxacin), Sulfonamiden oder Glycopeptiden (Vancomycin).

Ferner betrifft die Erfindung ein Verfahren zur Herstellung einer Verbindung der Formel (I), dadurch gekennzeichnet, dass

1. der Mikroorganismus *Aspergillus flavipes* ST003878 (DSM 15290) oder einer seiner Varianten und/oder Mutanten in einem Kulturmedium fermentiert wird, bis sich eine oder mehrere Verbindungen der Formel (I), in dem Kulturmedium anhäuft und
2. eine Verbindung der Formel (I) aus dem Kulturmedium isoliert wird, und
3. die Verbindung der Formel (I) gegebenenfalls derivatisiert und/oder in ein physiologisch verträgliches Salz überführt wird.

Vorzugsweise betrifft die Erfindung ein Verfahren zur Herstellung einer Verbindung der Formel (I), wobei in Schritt 1. die Verbindung der Formel (II) bei der Fermentierung entsteht, in Schritt 2. die Verbindung der Formel (II) isoliert wird, und in Schritt 3. gegebenenfalls zu einer Verbindung der Formel (I) derivatisiert und/oder in ein physiologisch verträgliches Salz überführt wird.

Das Verfahren umfasst die Kultivierung von *Aspergillus flavipes* ST003878 (DSM 15290) unter aeroben Bedingungen in einem eine Kohlenstoff-, eine Stickstoffquelle, anorganische Salze und gegebenenfalls Spurenelemente enthaltenden Kulturmedien. In diesem Kulturmedium bildet *Aspergillus flavipes* ST003878 (DSM 15290) ein Gemisch aus Verbindungen der Formel (I). Je nach Zusammensetzung des Kulturmediums kann der mengenmäßige Anteil eines oder mehrerer der erfindungsgemäßen Verbindungen variieren. Außerdem kann durch die Medienzusammensetzung die Synthese einzelner Verbindungen gesteuert werden.

Als Kohlenstoffquelle für die Fermentation eignen sich assimilierbare Kohlenhydrate und Zuckeralkohole, wie Glukose, Laktose, Saccharose, Glycerin, Stärke oder D-Mannit sowie kohlenhydrathaltige Naturprodukte, wie z.B. Malzextrakt oder Hefextrakt. Stickstoffhaltige Nährstoffe sind beispielsweise Aminosäuren; Peptide und Proteine sowie deren Abbauprodukte, beispielsweise Casein, Peptone oder Tryptone; Fleischextrakte; Hefeextrakte; Gluten; gemahlene Samen, beispielsweise von Mais, Weizen, Hafer, Bohnen, Soja oder der Baumwollpflanze; Destillationsrückstände der Alkoholherstellung; Fleischmehle; Hefeextrakte; Ammoniumsalze; Nitrate. Vorzugsweise ist die Stickstoffquelle ein oder mehrere synthetisch bzw. biosynthetisch gewonnene Peptide. Anorganische Salze sind beispielsweise Chloride, Carbonate, Sulfate oder Phosphate der Alkali- oder Erdalkalimetalle, Eisen, Zink, Kobalt und Mangan enthalten. Spurenelemente sind beispielsweise Kobalt und Mangan.

Vorzugsweise enthält das Kulturmedium Glukose, Stärke, Haferflocken und/oder Glycerin.

Die Bildung der erfindungsgemäßen Verbindungen verläuft besonders bevorzugt in einer Nährlösung, die etwa 0,05 bis 5 %, bevorzugt 2 bis 3 % Kartoffelstärke, und etwa 0,05 bis 3 %, bevorzugt 0.05 bis 1 % Hefeextrakt enthält. Die Angaben in Prozent sind jeweils bezogen auf das Gewicht der gesamten Nährlösung.

Die Kultivierung des Mikroorganismus erfolgt aerob, also beispielsweise submers unter Schütteln oder Rühren in Schüttelkolben oder Fermentern oder auf Festmedium, gegebenenfalls unter Einführen von Luft oder Sauerstoff. Sie kann in einem Temperaturbereich von etwa 15 bis 35°C, vorzugsweise bei etwa 20 bis 30°C, insbesonders bei 25°C durchgeführt werden. Der pH-Bereich sollte zwischen 3 und 10 liegen, vorzugsweise zwischen 4.5 und 6.5: Man kultiviert den Mikroorganismus unter diesen Bedingungen im allgemeinen über einen Zeitraum von 2 bis 30 Tagen, vorzugsweise 72 bis 360 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur; beispielsweise im Volumenverhältnis 1:10-1:100, überimpft werden. Die Vorkultur erhält man z. B., indem man das Mycel in eine Nährlösung überimpft und etwa 72 bis 360 Stunden, bevorzugt 96 bis 240 Stunden, wachsen läßt. Das Mycel kann beispielsweise erhalten werden, indem man den Stamm etwa 1 bis 20 Tage, bevorzugt 6 bis 10 Tage, auf einem festen oder flüssigen Nährboden, beispielsweise Hefe-Malz-Agar, Haferflocken-Agar oder Kartoffeldextrose-Agar, wachsen läßt.

Der Fermentationsverlauf und die Bildung der erfindungsgemäßen Verbindungen der Formel (I) kann entsprechend den dem Fachmann bekannten Methoden, wie z. B. durch Detektion der biologischen Aktivität in Bioassays, oder durch chromatographische Methoden wie Dünnschichtchromatographie (DC) oder HochJeistungsflüssigkeitschromatographie (HPLC) verfolgt werden.

Der Pilz *Aspergillus flavipes* ST003878 (DSM 15290) kann Verbindungen der Formel (I) beispielsweise durch eine Oberflächen- oder Standkultur auf festen Nährböden bilden. Feste Nährböden werden durch Zugabe zum Beispiel von Agar oder Gelatine zu wäßrigen Nährmedien hergestellt. Es ist aber auch möglich, die Verbindung der Formel (I) durch Fermentation des Pilzes *Aspergillus flavipes* ST003878 (DSM 15290) im Submersverfahren, d. h. in wäßriger Aufschlämmung zu gewinnen, wobei sich die Verbindung (I) gewöhnlich in der Zellmasse der Kultur befindet. Es ist deshalb zweckmäßig, die Fermentationslösung durch Filtration oder Zentrifugation zu trennen. Das Filtrat wird mit einem Adsorptionsharz als feste Phase extrahiert. Das Mycel und die Oberflächenkultur werden zweckmäßiger Weise mit einem mit Wasser mischbaren oder teilweise mischbaren organischen Lösungsmittel, beispielsweise einem ($C_1$-$C_4$)-Alkohol, vorzugsweise Methanol oder Propanol-2 extrahiert.

Die Extraktionen können in einem weiten pH-Bereich durchgeführt werden, es ist jedoch zweckmäßig, im neutralen oder schwach sauren Milieu, vorzugsweise zwischen pH 3 und pH 7 gegebenenfalls unter Zugabe eines Reduktionsmittels zu arbeiten. Die Extrakte können z. B. im Vakuum konzentriert und getrocknet werden.

Eine Methode der Aufreinigung der erfindungsgemäßen Verbindungen ist die Lösungsverteilung zwischen einer stationären und einer mobilen Phase in an sich bekannter Weise, beispielsweise durch Chromatographie an Adsorptionsharzen wie z. B. an Diaion® HP-20 (Mitsubishi Casei Corp., Tokyo), an Amberlite® XAD 7 (Rohm and Haas, USA) oder Amberchrom® CG, (Toso Haas, Philadelphia, USA). Geeignet sind darüber hinaus zahlreiche Reverse-, Phase Träger, z. B. $RP_8$ und $RP_{18}$, wie im Rahmen der Hochdruckflüssigkeits-Chromatographie (HPLC) allgemein bekannt.

Eine weitere Reinigungsmöglichkeit für das erfindungsgemäße Antibiotikum besteht in der Verwendung von sog. Normal-Phasen-Chromatographie-Trägern, wie z. B. Kieselgel oder $Al_2O_3$ oder anderen in an sich bekannter Weise.

Ein alternatives lsolierungsverfahren ist die Verwendung von Molekularsieben, wie z. B. Fractogel® TSK HW-40 (Merck, Deutschland) oder Sephadex® G-25 (Amersham Biosciences), in an sich bekannter Weise. Es ist darüber hinaus auch möglich, aus angereichertem Material das Biflavipin durch Kristallisation zu gewinnen. Geeignet hierzu sind z. B. organische Lösungsmittel und ihre Gemische, wasserfrei oder mit Wasserzusatz. Ein zusätzliches Verfahren zur Isolierung und Reinigung der erfindungsgemäßen Antibiotika besteht in der Verwendung von Anionenaustauschern, vorzugsweise im pH-Bereich von 4 bis 10 und Kationenaustauschern vorzugsweise im pH-Bereich von 2 bis 5. Besonders geeignet ist hierfür die Verwendung von Pufferlösungen, denen man Anteile von organischen Lösungsmitteln hinzugefügt hat.

Ein Isolat des Mikroorganismenstammes *Aspergillus flavipes* ST003878 wurde bei der Deutschen Sammlung von Mikroorganismen und Zellkulturen GmbH (DSMZ), Mascheroder Weg 1 B, 38124 Braunschweig, Deutschland nach den Regeln des Budapester Vertrages unter der Nummer DSM 15290 hinterlegt.

Der Pilz *Aspergillus flavipes* ST003878 (DSM 15290) besitzt ein weißes Substratmycel. Bei der Sporulation verfärbt sich das Mycel gelb bis braun. Bei Kulturen auf Czapek Agar Medium die etwa 10 Tage alt sind, werden reichlich gelb bis bräunliche Exudate gebildet. Die Konidien sind farblos rund mit einem Durchmesser von 2-3 $\mu$M. Die primären und die sekundären Sterigmata sind ähnlich lang (5-6 $\mu$m) und braun gefärbt.

Anstelle des Stammes *Aspergillus flavipes* ST003878 (DSM 15290) kann auch einer seiner Mutanten und/ oder Varianten eingesetzt werden, der eine der erfindungsgemäßen Verbindungen der Formel (I) herzustellen vermag.

Eine Mutante ist ein Mikroorganismus, in dem ein oder mehrere Gene des Genoms modifiziert wurden, wobei das Gen oder die Gene funktionell und vererbbar erhalten bleiben, die im Falle der vorliegenden Erfindung für die Fähigkeit des Organismus verantwortlich sind, eine oder mehrere der Verbindungen der Formel (i) zu produzieren.
Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS); 2-Hydroxy-4-methoxy-benzophenon (MOB) oder N-Methyl-N'-nitro-N-nitrosoguanidin (MNNG) erzeugt werden, oder wie von Brock et al. in "Biology of Microorganism", Prentice Hall, Seite 238-247 (1984) beschrieben.

Eine Variante ist ein Phenotyp des Mikroorganismus. Mikroorganismen haben die Fähigkeit, sich an ihre Umgebung anzupassen und zeigen daher ausgeprägte physiologische Flexibilität. Bei der phenotypischen Anpassung sind alle Zellen des Mikroorganismus involviert, wobei die Art der Veränderung nicht genetisch konditioniert ist und unter veränderten Bedingungen reversibel ist (H. Stolp, Microbial ecology: organismus, habitats, activities. Cambridge University Press, Cambridge, GB, Seite 180, 1988).

Das Screening nach Mutanten und/oder Varianten, die ein oder mehrere der erfindungsgemäßen Verbindungen synthetisieren, erfolgt nach folgendem Schema:

- Lyophilisierung des Fermentationsmediums;
- Extraktion des Lyophilisats mit einem organischen Lösungsmittel
- Extraktion der Verbindung aus dem Kulturfiltrat mit Festphasen
- Analytik mittels HPLC, DC oder durch Testen der biologischen Wirksamkeit.

Die oben beschriebenen Fermentationsbedingungen gelten für *Aspergillus flavipes* ST003878 (DSM 15290) sowie für Mutanten und/oder Varianten davon.

Derivatisierungen werden wie folgt durchgeführt: Hydroxygruppen der Verbindung der Formel (I) ($R_3$ bis $R_7$ unabhängig voneinander gleich H) können mit einer aktivierten Säure, vorzugsweise einem Säurechlorid Cl-C(O)-($C_1$-$C_6$)-Alkyl verestert werden ($R_3$ bis $R_7$ unabhängig voneinander gleich -C(O)-($C_1$-$C_6$)-Alkyl). Hydroxygruppen können ferner verethert werden durch beispielsweise Umsetzung mit einem ($C_1$-$C_6$)-Alkylhalogenid im saurem Medium oder durch Umsetzung mit einer Trimethylsilyl-diazo-($C_1$-$C_6$)-Alkylverbindung, vorzugsweise mit Trimethylsilyl-diazomethan. Eine Oxidation einer phenylischen Aldehydfunktion ($R^1$ und/oder $R^2$ gleich H) zu einer Säurefunktion erfolgt beispielsweise mittels Manganoxid oder mittels Jones-Reagenz (schwefelsaures Chrom(VI)-oxid). Die dabei entstandene Säurefunktion kann unter Steglich-Bedingungen mittels 4-Dimethylaminopyridin (4-DMAP) oder im sauren Medium mit einem ($C_1$-$C_6$)-Alkohol verestert werden. Die genannten Derivatisierungen sind Beispiele für an sich bekannte Methoden und unter anderem beschrieben in J. March in Advanced Organic Chemistry, John Wiley & Sons, 4$^{th}$ Edition, 1992. Um Umsetzungen selektiv durchzuführen, kann es vorteilhaft sein, vor der Reaktion geeignete, in an sich bekannter Weise, Schutzgruppen einzuführen. Die Schutzgruppen werden nach der Reaktion abgespaltet, und anschließend wird das Reaktionsprodukt gereinigt.

Die erfindungsgemäßen Verbindungen sind starke Hemmstoffe der bakterielle N-Acetyl-glucosamin-1-phosphat-uridyltransferase (GlmU), sie eignet sich daher zur Behandlung und/oder Prophylaxe von Erkrankungen, die durch bakterielle Infektionen oder Mykosen verursacht werden.

Die Inhibition der GlmU kann in einem biochemischen Test nach folgendem Verfahren bestimmt werden:

Der Assay wurde im 384er Plattenformat durchgeführt. Das Reaktionsvolumen betrug 40 μl pro Test. Die Reaktionsmischung enthielt 10 μl Testsubstanz und 15 μl Substratlösung bestehend aus AcetylCoA und D-Glucosamin-1-Phosphat. Durch Zugabe von 15 μl GlmU-Enzymlösung wurde die enzymatische Reaktion gestartet. Nach 60 Minuten Inkubation bei 30°C wurde die Reaktion durch Zugabe von 10 μl Entwicklerreagenz (DTNB gelöst in Guanidin) gestoppt. Anschließend wurden die Platten zentrifugiert (1 min, 1000 rpm) und für weitere 45 Minuten bei Raumtemperatur aufbewahrt, bevor die Absorption bei 405 nm in einem Photometer gemessen wurde. Um die inhibitorische Aktivität der Testverbindungen berechnen zu können, wurden auf jeder Platte Positivkontrollen (Signal mit GlmU) und Negativkontrollen (Signal ohne GlmU) mitgeführt. Um falsch-positive Proben auszuschließen, die durch nichtspezifische Substanzeffekte verursacht werden könnten (z. B. Farbe) wurden parallel zu den Testplatten Blankplatten mitgeführt, die die Substanzen, aber kein GlmU enthielten. Nach Blankbereinigung wurden die inhibitorischen Aktivitäten der Substanzen gemäß folgender Formel berechnet:

$$\text{Inhibition (\%)} = 100 \times [1 - (OD_{405\,nm}\,\text{Substanz} / OD_{405\,nm}\,\text{Positivkontrolle})]$$

Für die Hemmwirkung der Verbindung der Formel (II) am Enzym N-Acetyl-glucosamin-1-phosphat-uridyltransferase (GlmU) wurde eine Hemmkonstante $IC_{50}$ von 1 μM bestimmt.

Gegenstand der Erfindung ist daher ferner die Verwendung einer Verbindung der Formel (I), vorzugsweise einer Verbindung der Formel (II), oder eines physiologisch verträglichen Salzes davon zur Herstellung eines Arzneimittels in der Human- oder Tiermedizin, insbesondere zur Herstellung eines Arzneimittels zur Behandlung und/oder Prophylaxe von bakteriellen Infektionskrankheiten und/oder von Mykosen.

Des weiteren betrifft die vorliegende Erfindung ein Arzneimittel mit einem Gehalt an mindestens einer erfindungsgemäßen Verbindung der Formel (I), vorzugsweise mit einem Gehalt an der Verbindung der Formel (II).

Das besagte Arzneimittel wird durch Mischung von mindestens einer Verbindung der Formel (I) mit einem oder mehreren pharmakologisch geeigneten Hilfs- oder Trägerstoffen hergestellt und in eine geeignete Darreichungsform gebracht.

Die erfindungsgemäßen Arzneimittel können oral oder parenteral verabreicht werden, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise pharmakologisch geeignete Träger- oder Hilfsstoffe wie Spreng-, Binde-, Überzugs-, Quellungs-, Gieit- oder Schmiermittel, Geschmacksstoffe, Süßungsmittel oder Lösungsvermittler Verwendung finden, beispielsweise Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa Wasser, Alkohole, Glycerin und mehrwertige Alkohole.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Als zweckmäßige Dosierung werden 0.1 - 1000, vorzugsweise 0.2 - 100 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die folgenden Beispiele sollen der näheren Erläuterung der Erfindung dienen, ohne die Breite der Erfindung in irgendeiner Weise zu begrenzen.

Prozentangaben beziehen sich auf das Gewicht. Mischungsverhältnisse bei Flüssigkeiten beziehen sich auf das Volumen, wenn keine anderen Angaben gemacht wurden.

Beispiel 1: Herstellung einer Glycerinkultur von *Aspergillus flavipes* ST003878 (DSM 15290)

30 mL Nährlösung (Malzextrakt 2.0%, Hefeextrakt 0.2 %, Glucose 1.0 % $(NH_4)_2HPO_4$ 0.05 %, pH 6.0) in einem sterilen 100 mL Erlenmeyerkolben wurden mit dem Stamm *Aspergillus flavipes* ST003878 (DSM 15290), beimpft und 6 Tage bei 25°C und 140 UpM auf einer rotierenden Schüttelmaschine inkubiert. 1.5 mL dieser Kultur wurden anschließend mit 2.5 mL 80%igem Glycerin verdünnt und bei -135°C gelagert.

Beispiel 2: Herstellung einer Vorkultur im Erlenmeyerkolben von *Aspergillus flavipes* ST003878 (DSM 15290)

100 mL Nährlösung (Malzextrakt 2.0%, Hefeextrakt 0.2 %, Glucose 1.0 % $(NH_4)_2HPO_4$ 0.05 %, pH 6.0) in einem sterilen 300 mL Erlenmeyerkolben werden mit dem Stamm *Aspergillus flavipes* ST003878 (DSM 15290), beimpft und 4 Tage bei 25° C und 140 UpM auf einer rotierenden Schüttelmaschine inkubiert. 2 mL dieser Vorkultur werden anschließend für die Herstellung der Hauptkulturen beimpft.

Beispiel 3: Fermentation von *Aspergillus flavipes* ST003878 (DSM 15290) im Erlenmeyerkolben

Je 100 mL Nährlösung (Potato Dextrose Broth 2.4%, Hefeextrakt 0.2 %, pH 5.2) in sterilen 300 mL Erlenmeyerkolben werden mit dem Stamm *Aspergillus flavipes* ST003878 (DSM 15290), beimpft und 11 Tage bei 25°C und 140 UpM auf einer rotierenden Schüttelmaschine inkubiert. 300 mL dieser Vorkultur werden anschließend für die Herstellung der Hauptkulturen beimpft.

Beispiel 4: Fermentation von *Aspergillus flavipes* ST003878 (DSM 15290) im Fermenter

Ein 10 L Fermenter wird unter folgenden Bedingungen betrieben:

| | |
|---|---|
| Nährmedium: | 24 g/L Potato Dextrose Broth |
| | 2 g/L Hefeextrakt pH 5.2 (vor der Sterilisation) |
| Inkubationszeit: | 166 Stunden |
| Inkubationstemperatur: | 28°C |

(fortgesetzt)

| | |
|---|---|
| Rührergeschwindigkeit: | 180 UpM |
| Belüftung: | 16 L $Min^{-1}$ |

Durch wiederholte Zugabe von ethanolischer Polyollösung wurde die Schaumbildung unterdrückt. Das Produktionsmaximum wurde nach ca. 160 Stunden erreicht.

Beispiel 5: Isolierung der Verbindung der Formel (II)

25 Liter Kulturlösung, gewonnen nach Beispiel 4, enthielten 90 mg der Verbindung der Formel (II). Die Kulturbrühe wurde filtriert und das Mycel (1.65 kg) mit 10 Liter Propanol-2 extrahiert. Die klare alkoholische Phase wurde im Vakuum auf 2 L eingeengt und nach Zugabe von 1 g Ascorbinsäure als Antioxidanz auf eine 785 mL fassende, mit dem Adsorptionsharz MCI Gel® CHP20P gefüllte Säule aufgetragen. Säulenmaße: Breite x Höhe: 10 cm x 25 cm. Eluiert wurde mit einem Lösungsmittel-Gradienten von 5% Acetonitril in Wasser bis 100% Acetonitril und einem Säulendurchfluß von 15 Liter pro Stunde. Es wurden Fraktionen je 2.5 Liter Inhalt aufgefangen. Die Fraktionen 7 bis 9, die die Verbindung der Formel (II) enthielten, wurden durch HPLC-Analysen überprüft, gesammelt und im Vakuum konzentriert. Der pH-Wert der vom Isopropanol weitgehend befreiten Lösung wurde mit auf 4.2 eingestellt und erneut auf eine MCI Gel® CHP20P-Säule (490 mL, Säulenmaße: 5 cm x 25 cm) aufgetragen. Die Elution erfolgte im Gradientenverfahren von 0.01 % Ammoniumformiat (pH 4.2) nach 100% Acetonitril. Bei einem Säulendurchfluß von 40 mL pro Minute wurden Fraktionen von 200 mL Inhalt abgenommen. Die Fraktionen 12 und 13 enthielten die angereicherte Verbindung der Formel (II). Sie wurden vereinigt, unter Vakuum eingeengt und zur weiteren chromatographischen Reinigung auf einer Luna 10µ C18 phenomenex®-Säule getrennt (Säulenmaße: 21 mm x 250 mm). Als Elutionsmittel diente 0.025% Trifluoressigsäure und 0% bis 100% Acetonitril bei einem Durchfluß von 25 mL/Minute. Die Fraktionsgröße betrug 50 mL. Die Fraktion 14 enthielt die stark angereicherte Verbindung der Formel (II), sie wurde im Vakuum leicht eingeengt und bei +4° C stehen gelassen. Die zitronengelbe Verbindung der Formel (II) kristallisierte aus, wurde abfiltriert, getrocknet und unter Argon abgefüllt (20 mg).

Beispiel 6: Hochdruckflüssigkeitschromatographie (HPLC) der Verbindung der Formel (II)

| | |
|---|---|
| Säule: | YMC-PRO 18®, 120°A, 250-4.4, mit Vorsäule, |
| Mobile Phase: | A: 5% Acetonitril in 0.02% Trifluoressigsäure, 2 Minuten, |
| | B: 100% Acetonitril, |
| Gradient: | Von A nach B in 18 Minuten. |
| Flußgeschwindigkeit: | 1 mL pro Minute, |

Detektion durch UV-Absorption bei 210 nm.

Für die Verbindung der Formel (II) wurde eine Retentionszeit von 13.9 Minuten gefunden.

Beispiel 7: Charakterisierung der Verbindung der Formel (II)

Durch Elektronenspray-Ionisations Massenspektrometrie (ESI-MS) im negativem Modus wurde eine Molmasse (M - H) von 357.0673 gefunden, im positivem Modus ein Molekulargewicht (M + H) von 359.0811, entsprechend einer Summenformel $C_{18}H_{14}O_8$ und einem Molekulargewicht von 358.31.

Die physikalisch-chemischen sowie spektroskopischen Eigenschaften der Verbindung (II) lassen sich wie folgt zusammenfassen:

Aussehen: Zitronengelbe, in mittelpolaren und polaren organischen Lösungsmitteln lösliche kristalline Substanz. Stabil in neutralem und mild saurem Milieu, jedoch unbeständig in stark-saurer und alkalischer Lösung und unter Sauerstoffeinwirkung.

Summenformel: $C_{18}H_{14}O_8$

Molekulargewicht: 358.31

UV-Maxima: 220, 245, 307 und 365 nm in Wasser/Acetonitril (1:1) bei pH 2.

Tabelle 1: Chemische Verschiebungen der Verbindung (II) in DMSO bei 300 K.

| | $^{1}H$ | $^{13}C$ |
|---|---|---|
| 2 | - | 151.57 |
| 3 | 7.47 | 108.94 |
| 3a | - | 122.83 |
| 4 | - | 117.21 |
| 5 | - | 127.54 |
| 5-Me | 2.58 | 11.03 |
| 6 | - | 140.91 |
| 6-OH | breit | - |
| 7 | - | 136.42 |
| 7-OH | breit | - |
| 7a | - | 142.35 |
| 8 | 10.42 | ~190.2 (breit) |
| 9 | - | 124.84 |
| 10 | - | 112.61 |
| 11 | - | 150.17 |
| 11-OH | ~12.1 (breit) | - |
| 12 | - | 132.68 |
| 12-OH | breit | - |
| 13 | - | 151.52 |
| 13-OH | breit | - |
| 14 | - | 118.72 |
| 14-Me | 2.01 | 12.66 |
| 15 | 9.48 | 194.74 |

Beispiel 8: Methylierung der Verbindung der Formel (II)

12 mg der Verbindung der Formel (II), gewonnen nach Beispiel 5, wurden in 3 mL Methanol gelöst und mit 1 mL (Trimethylsilyl)-diazomethan [2 M Lösung in Hexan, Aldrich] versetzt. Der Reaktionsansatz wurde bei Raumtemperatur stehen gelassen, und der Verlauf der Reaktion mit HPLC verfolgt. Nachdem die eingesetzte Verbindung der Formel (II) vollständig umgesetzt war, wurde die Reaktion durch Zugabe von Wasser und Ein-engen im Vakuum abgebrochen. Die entstandenen Methylierungsprodukte wurden chromatographisch auf einer Luna 5 μ RP18 phenomenex®-Säule mit 0.02% Trifluoressigsäure und Acetonitril im Gradientenverfahren aufgereinigt. Nach der Gefriertrocknung der reinen Fraktionen erhielt man durch HPLC (Bedingungen wie in Beispiel 5):
2 mg Verbindung der Formel (III), Monomethylether, Retentionszeit 15.4 Minuten, 2.7 mg Verbindung der Formel (IV), Dimethylether, Retentionszeit 16.9 Minuten, 1.9 mg Verbindung der Formel (V), Trimethylether, Retentionszeit 19.4 Minuten.

Beispiel 9: Charakterisierung der Verbindung der Formel (III)

Die Verbindung der Formel (III) ergab im ESI-MS im positiven Modus einen Molekularpeak von 373.0 (M + H) sowie im negativem Modus 371.0 (M - H), entsprechend dem MG = 372 und der Summenformel $C_{19}H_{16}O_8$.

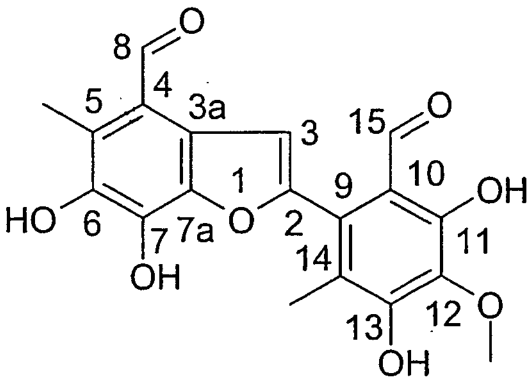

Tabelle 2: Chemische Verschiebungen der Verbindung (III) in DMSO bei 300 K.

| | $^{1}H$ | $^{13}C$ |
|---|---|---|
| 2 | - | 150.95 |
| 3 | 7.51 | 109.16 |
| 3a | - | 122.65 |
| 4 | - | 117.28 |
| 5 | - | 127.75 |
| 5-Me | 2.58 | 11.06 |
| 6 | - | 141.03 |
| 6-OH | breit | - |
| 7 | - | 136.47 |
| 7-OH | breit | - |
| 7a | - | 142.45 |
| 8 | 10.42 | 190.24 |
| 9 | - | 129.11 |
| 10 | - | 112.98 |
| 11 | - | 154.87 |
| 11-OH | 12.22 | - |
| 12 | - | 134.76 |
| 12-OMe | 3.83 | 60.28 |
| 13 | - | 155.68 |
| 13-OH | breit | - |
| 14 | - | 119.05 |
| 14-Me | 2.01 | 12.66 |
| 15 | 9.49 | 194.73 |

Beispiel 10: Charakterisierung der Verbindung der Formel (IV)

Die Verbindung der Formel (IV) ergab im ESI-MS im positivem Modus einen Molekularpeak von 387.1 (M + H) sowie im negativem Modus 385.0 entsprechend dem MG = 386 und der Summenformel $C_{20}H_{18}O_8$.

Tabelle 3: Chemische Verschiebungen der Verbindung (IV) in DMSO bei 300 K.

| | $^1H$ | $^{13}C$ |
|---|---|---|
| 2 | - | 151.51 |
| 3 | 7.51 | 108.72 |
| 3a | - | 122.93 |
| 4 | - | 119.83 |
| 5 | - | 127.10 |
| 5-Me | 2.58 | 10.86 |
| 6 | - | 143.62 |
| 6-OH | 9.21 | - |
| 7 | - | 137.18 |
| 7-OMe | 4.15 | 60.44 |
| 7a | - | 143.74 |
| 8 | 10.48 | 190.88 |
| 9 | - | 122.97 |
| 10 | - | 115.84 |
| 11 | - | 150.54 |
| 11-OH | 11.70 | - |
| 12 | - | 139.24 |
| 12-OH | 9.68 | - |
| 13 | - | 151.69 |
| 13-OMe | 3.87 | 59.90 |
| 14 | - | 123.98 |
| 14-Me | 2.08 | 12.80 |
| 15 | 9.72 | 195.45 |

Beispiel 11: Charakterisierung der Verbindung der Formel (V)

Die Verbindung der Formel (V) ergab im ESI-MS im positivem Modus einen Molekularpeak von 401.1 (M + H) und im negativem Modus 399.0 (M - H), entsprechend MG = 400 und der Summenformel $C_{21}H_{20}O_8$.

Tabelle 4: Chemische Verschiebungen der Verbindung (V) in DMSO bei 300 K.

| | $^1H$ | $^{13}C$ |
|---|---|---|
| 2 | - | 150.90 |
| 3 | 7.56 | 108.95 |
| 3a | - | 122.73 |
| 4 | - | 119.91 |
| 5 | - | 127.28 |
| 5-Me | 2.58 | 10.85 |
| 6 | - | 143.74 |
| 6-OH | 9.25 | - |

(fortgesetzt)

| | $^1H$ | $^{13}C$ |
|---|---|---|
| 7 | - | 137.18 |
| 7-OMe | 4.14 | 60.45 |
| 7a | - | 143.82 |
| 8 | 10.48 | 190.88 |
| 9 | - | 127.83 |
| 10 | - | 116.20 |
| 11 | - | 155.63 |
| 11-OH | 11.82 | - |
| 12 | - | 140.70 |
| 12-OMe | 3.88 | 60.53 |
| 13 | - | 156.89 |
| 13-OMe | 3.98 | 60.74 |
| 14 | - | 123.77 |
| 14-Me | 2.06 | 12.85 |
| 15 | 9.73 | 195.00 |

**Patentansprüche**

**1.** Verbindung der Formel (I)

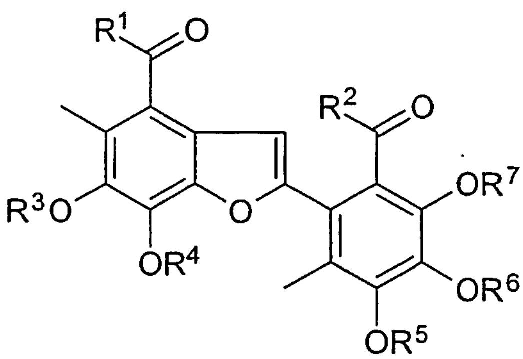

(I)

wobei
$R^1$ und $R^2$ unabhängig voneinander H, OH oder -O-($C_1$-$C_6$)-Alkyl, und $R^3$, $R^4$, $R^5$, $R^6$ und $R^7$ unabhängig voneinander H, -($C_1$-$C_6$)-Alkyl oder
-C(O)-($C_1$-$C_6$)-Alkyl bedeuten,
oder ein physiologisch verträgliches Salz der Verbindung der Formel (I).

**2.** Verbindung der Formel (I) gemäß Anspruch 1, wobei $R^1$ und $R^2$ gleich H sind.

**3.** Verbindung der Formel (I) gemäß einem der Ansprüche 1 oder 2, wobei $R^3$, $R^4$, $R^5$, $R^6$ und $R^7$ unabhängig voneinander gleich H oder Methyl sind.

**4.** Verbindung der Formel (I) gemäß einem der Ansprüche 1 bis 3, **gekennzeichnet durch** eine Verbindung der Formel

(II)

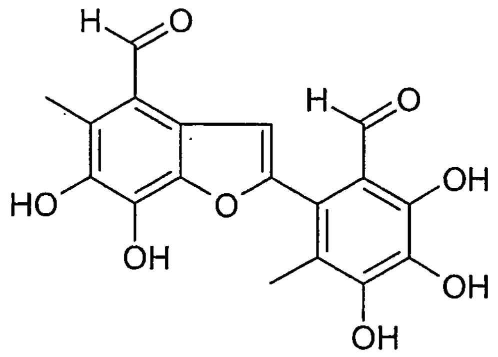

(II).

5. Verbindung der Formel (i) gemäß einem der Ansprüche 1 bis 3, **gekennzeichnet durch** eine Verbindung der Formel (III)

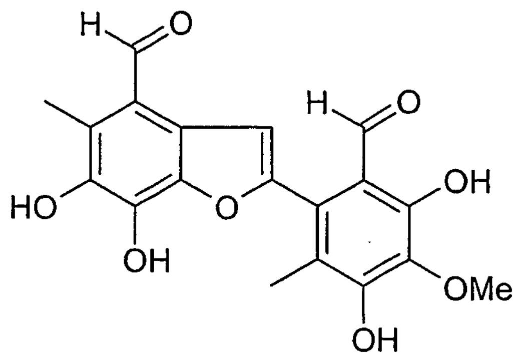

(III).

6. Verbindung der Formel (I) gemäß einem der Ansprüche 1 bis 3, **gekennzeichnet durch** eine Verbindung der Formel (IV)

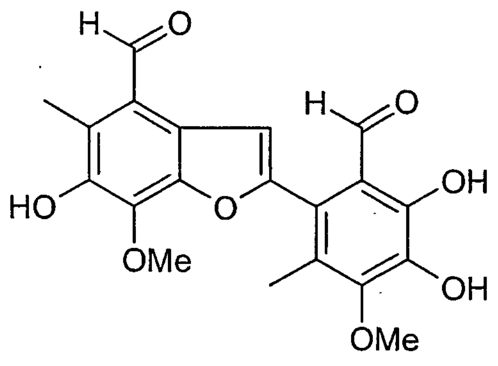

(IV).

7. Verbindung der Formel (I) gemäß einem der Ansprüche 1 bis 3, **gekennzeichnet durch** eine Verbindung der Formel (V)

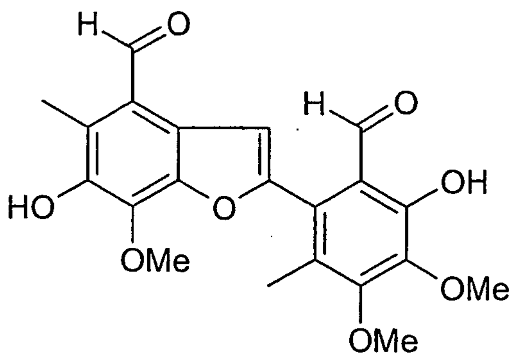

(V).

**8.** Verfahren zur Herstellung einer Verbindung der Formel (I) gemäß einem der Ansprüche 1 bis 7, **dadurch gekennzeichnet, dass**

1. der Mikroorganismus *Aspergillus flavipes* ST003878 (DSM 15290) oder einer seiner Varianten und/oder Mutanten in einem Kulturmedium fermentiert wird, bis sich eine oder mehrere Verbindungen der Formel (I), in dem Kulturmedium anhäuft und
2. eine Verbindung der Formel (I) aus dem Kulturmedium isoliert wird, und
3. die Verbindung der Formel (I) gegebenenfalls derivatisiert und/oder in ein physiologisch verträgliches Salz überführt wird.

**9.** Verfahren gemäß Anspruch 8, wobei bei der Fermentierung in Schritt 1. die Verbindung der Formel (II) entsteht, in Schritt 2. die Verbindung der Formel (II) isoliert wird, und in Schritt 3. gegebenenfalls zu einer Verbindung der Formel (I) derivatisiert und/oder in ein physiologisch verträgliches Salz überführt wird.

**10.** Verwendung einer Verbindung der Formel (I) gemäß einem der Ansprüche 1 bis 7 oder eines physiologisch verträglichen Salzes davon zur Herstellung eines Arzneimittels in der Human- oder Tiermedizin.

**11.** Verwendung einer Verbindung der Formel (I) gemäß Anspruch 10 zur Herstellung eines Arzneimittels zur Behandlung und/oder Prophylaxe von bakteriellen Infektionskrankheiten und/oder von Mykosen.

**12.** Arzneimittel mit einem Gehalt an mindestens einer erfindungsgemäßen Verbindung der Formel (I) gemäß einem der Ansprüche 1 bis 7 oder eines physiologisch verträglichen Salzes davon.

**13.** Verfahren zur Herstellung eines Arzneimittel gemäß Anspruch 12, wobei mindestens eine Verbindung der Formel (I) mit einem oder mehreren pharmakologisch geeigneten Hilfs- oder Trägerstoff vermischt wird.

**14.** Mikroorganismus *Aspergillus flavipes* ST003878 (DSM 15290).

## Claims

**1.** Compound of the formula (I)

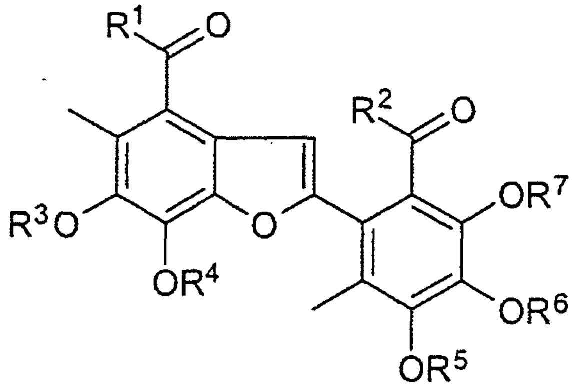

(I)

wherein
$R^1$ and $R^2$ are, independently of each other, H, OH or -O-($C_1$-$C_6$)-alkyl, and
$R^3$, $R^4$, $R^5$, $R^6$ and $R^7$ are, independently of each other, H, - ($C_1$-$C_6$) -alkyl or -C(O)-($C_1$-$C_6$)-alkyl,
or a physiologically tolerated salt of a compound of the formula (I).

**2.** Compound of the formula (I) according to Claim 1, wherein $R^1$ and $R^2$ are H.

**3.** Compound of the formula (I) according to Claim 1 or 2, wherein $R^3$, $R^4$, $R^5$, $R^6$ and $R^7$ are, independently of each other, H or methyl.

**4.** Compound of the formula (I) according to one of Claims 1 to 3, **characterized by** a compound of the formula (II)

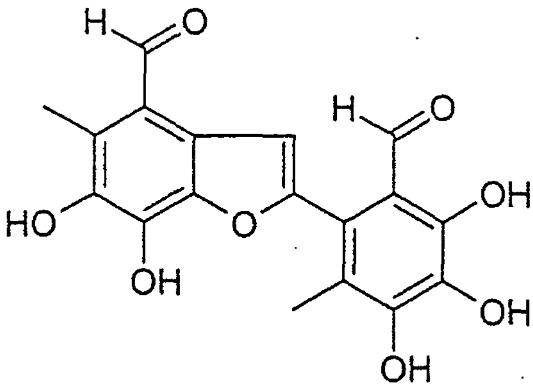

(II).

**5.** Compound of the formula (I) according to one of Claims 1 to 3, **characterized by** a compound of the formula (III)

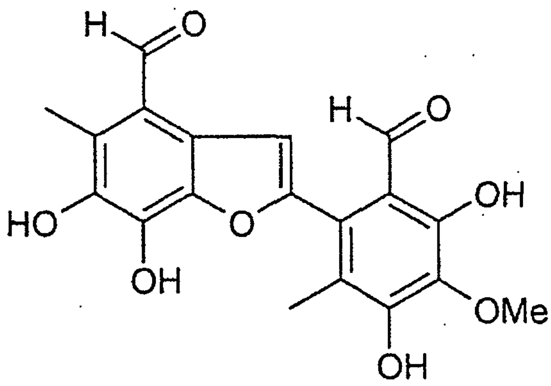

(III).

6. Compound of the formula (I) according to one of Claims 1 to 3, **characterized by** a compound of the formula (IV)

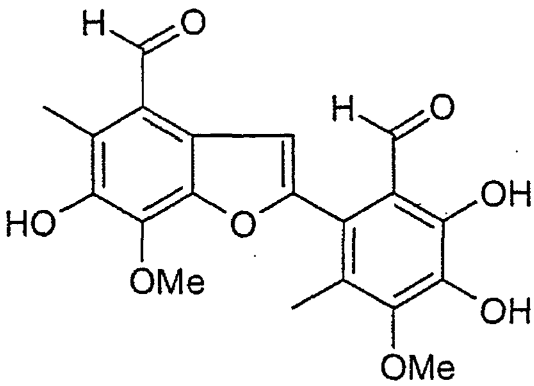

(IV).

7. Compound of the formula (I) according to one of Claims 1 to 3, **characterized by** a compound of the formula (V)

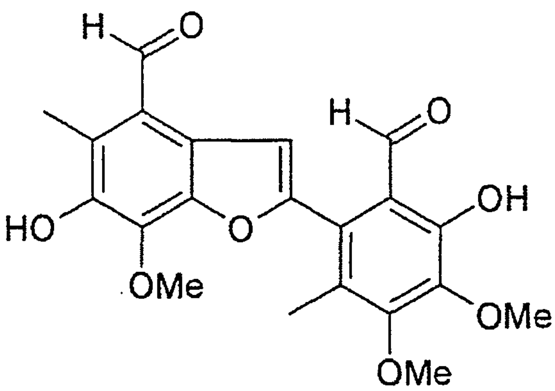

(V).

8. Process for preparing a compound of the formula (I) according to one of Claims 1 to 7, **characterized in that** it comprises

1. fermenting the microorganism *Aspergillus flavipes* ST003878 (DSM 15290), or one of its variants and/or mutants, in a culture medium until one or more compounds of the formula (I) accrues in the culture medium, and

2. isolating a compound of the formula (I) from the culture medium, and
3. where appropriate derivatizing the compound of the formula (I) and/or converting it into a physiologically tolerated salt.

**9.** Process according to Claim 8, wherein, during the fermentation, the compound of the formula (II) is formed in step 1, the compound of the formula (II) is isolated in step 2 and, in step 3, it is derivatized, where appropriate, to give a compound of the formula (I) and/or converted into a physiologically tolerated salt.

**10.** Use of a compound of the formula (I) according to one of Claims 1 to 7, or of a physiologically tolerated salt thereof, for producing a pharmaceutical in human or animal medicine.

**11.** Use of a compound of the formula (I) according to Claim 10 for producing a pharmaceutical for the treatment and/or prophylaxis of bacterial infectious diseases and/or mycoses.

**12.** Pharmaceutical having a content of at least one compound of the formula (I) according to the invention according to one of Claims 1 to 7, or of a physiologically tolerated salt thereof.

**13.** Process for producing a pharmaceutical according to Claim 12, which comprises mixing at least one compound of the formula (I) with one or more pharmacologically suitable auxiliary substances or carrier substances.

**14.** Microorganism *Aspergillus flavipes* ST003878 (DSM 15290).

## Revendications

**1.** Composé de formule (I)

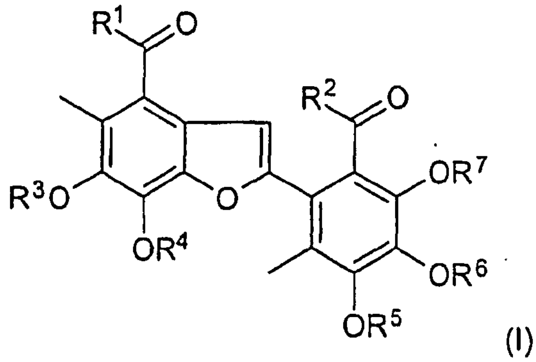

(I)

où
$R^1$ et $R^2$ signifient, indépendamment l'un de l'autre, H, OH ou -O-($C_1$-$C_6$)-alkyle, et
$R^3$, $R^4$, $R^5$, $R^6$ et $R^7$ signifient, indépendamment l'un de l'autre, H, - ($C_1$-$C_6$)-alkyle ou -C (O) - ($C_1$-$C_6$) -alkyle,
ou un sel physiologiquement acceptable du composé de formule (I).

**2.** Composé de formule (I) selon la revendication 1, où $R^1$ et $R^2$ représentent H.

**3.** Composé de formule (I) selon l'une quelconque des revendications 1 ou 2, où $R^3$, $R^4$, $R^5$, $R^6$ et $R^7$ représentent, indépendamment l'un de l'autre, H ou méthyle.

**4.** Composé de formule (I) selon l'une quelconque des revendications 1 à 3, **caractérisé par** un composé de formule (II)

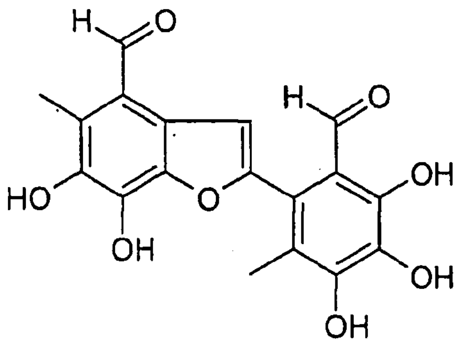

(II).

5. Composé de formule (I) selon l'une quelconque des revendications 1 à 3, **caractérisé par** un composé de formule (III)

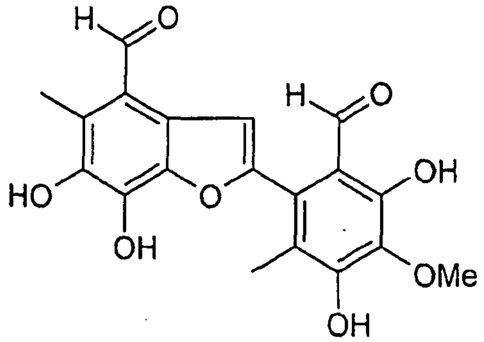

(III).

6. Composé de formule (I) selon l'une quelconque des revendications 1 à 3, **caractérisé par** un composé de formule (IV)

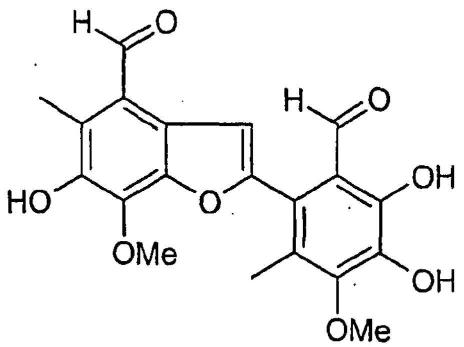

(IV).

7. Composé de formule (I) selon l'une quelconque des revendications 1 à 3, **caractérisé par** un composé de formule (V)

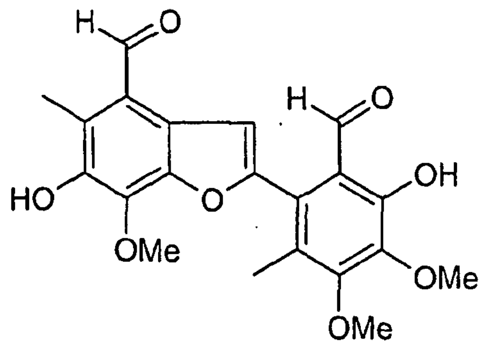

(V).

**8.** Procédé pour la préparation d'un composé de formule (I) selon l'une quelconque des revendications 1 à 7, **caractérisé en ce que**

1. le microorganisme Aspergillus flavipes ST003878 (DSM 15290) ou un de ses variants et/ou un de ses mutants est fermenté dans un bouillon de culture jusqu'à ce qu'un ou plusieurs composés de formule (I) s'accumulent dans le bouillon de culture et
2. un composé de formule (I) est isolé du bouillon de culture et
3. le composé de formule (I) est le cas échéant dérivé et/ou transformé en un sel physiologiquement acceptable.

**9.** Procédé selon la revendication 8, dans lequel le composé de formule (II) se forme lors de la fermentation dans l'étape 1, le composé de formule (II) est isolé dans l'étape 2 et, le cas échéant, dérivé en un composé de formule (I) et/ou transformé en un sel physiologiquement acceptable dans l'étape 3.

**10.** Utilisation d'un composé de formule (I) selon l'une quelconque des revendications 1 à 7 ou d'un sel physiologiquement acceptable de celui-ci pour la préparation d'un médicament en médecine humaine ou animale.

**11.** Utilisation d'un composé de formule (I) selon la revendication 10 pour la préparation d'un médicament pour le traitement et/ou la prophylaxie de maladies infectieuses bactériennes et/ou de mycoses.

**12.** Médicament présentant une teneur en au moins un composé selon l'invention de formule (I) selon l'une quelconque des revendications 1 à 7 ou un sel physiologiquement acceptable de celui-ci.

**13.** Procédé pour la préparation d'un médicament selon la revendication 12, dans lequel au moins un composé de formule (I) est mélangé avec un ou plusieurs adjuvants ou supports pharmacologiquement acceptables.

**14.** Microorganisme Aspergillus flavipes ST003878 (DSM 15290).